# EUROPEAN PATENT APPLICATION

(11) **EP 3 881 807 A1**
(43) Date of publication of application: **22.09.2021**
(21) Application number: 19883597.7
(22) Date of filing: 13.11.2019
(51) Int. Cl.: A61F 2/966, A61M 25/092

(54) **MEDICAL RETENTION INSTRUMENT CONVEYANCE DEVICE AND MEDICAL RETENTION INSTRUMENT CONVEYANCE DEVICE WITH RETENTION INSTRUMENT**

(30) Priority: 13.11.2018 JP 2018212994; 13.11.2018 JP 2018212995
(71) Applicant: Sumitomo Bakelite Co.Ltd., Shinagawa-ku Tokyo 140-0002 (JP)
(72) Inventor: KANEMASA Kenichi, Akita-shi, Akita 011-8510 (JP); ASAI Hideaki, Akita-shi, Akita 011-8510 (JP)
(74) Representative: Ter Meer Steinmeister & Partner
(86) International application number: PCT/JP2019/044509
(87) International publication number: WO 2020/100935

(57) **Abstract**

A transporting apparatus including: a sheath that holds a stent graft; an operating line slidably inserted into the sheath and whose distal side is connected to a tip portion of the sheath; an operating lever that is connected to a proximal side of the operating line and bends the tip portion of the sheath by towing the operating line; a holding shaft capable of restricting movement of the stent graft to the proximal side; and a movement mechanism that changes a traction force of the sheath when relative positions of the holding shaft and the sheath are changed so that the sheath is relatively located on the proximal side.

## Description

### [Technical Field]

The present invention relates to an indwelling medical device transporting apparatus for delivering an indwelling device such as a stent or a stent graft to the inside of a lumen of a living body; and an indwelling medical device transporting apparatus with an indwelling device.

Priority is claimed on Japanese Patent Application No. 2018-212994 and Japanese Patent Application No. 2018-212995, filed November 13, 2018, the contents of which are incorporated herein by reference.

### [Background Art]

Stents are used by being indwelled (i.e., placed and left to remain) in areas of constriction, occlusion, adhesion (hereinafter, may be referred to as "constricted area and the like") or the like generated in a lumen of a living body. Stents are generally formed as expandable, mesh-like small metal tubes. Bare metal stents to which no drug is applied and drug-eluting stents from which the applied drug is slowly released have been known.

A stent graft is a tubular indwelling device produced by integrating an artificial blood vessel (graft) with a metal stent formed into mesh-like network. The stent graft is used by being placed to remain inside an aortic aneurysm and the like in the abdomen or chest in addition to a constricted area and the like. According to the stent graft, blood can flow inside the graft, blood pressure is not applied to the aortic aneurysm outside the graft, and rupture of the aortic aneurysm can be prevented.

Stents are broadly classified into balloon expandable stents and self expandable stents. In a balloon expandable type, a stent folded into a tubular shape does not expand by itself, but expands by inflating a balloon provided in a balloon catheter and is attached to the inner surface of an aneurysm. On the other hand, a self expandable stent is made of a superelastic metal and is constrained in a contracted state compressed to a small diameter. By releasing this constraint, the stent expands by itself.

Stents and stent grafts are attached to a distal portion of a long and flexible tubular main body called a catheter shaft or sheath and introduced into the lumen of a living body.

For example, Patent Document 1 describes a stent graft placing device that delivers a self expandable stent graft into a lumen of a living body.

A sheath to which the stent graft of Patent Document 1 is attached is pre-shaped (shaped) into a curved shape. An operator can place the stent graft installed inside the sheath at the desired position by inserting the sheath along a guide wire that runs from the femoral region to the blood vessel near the shoulder.

Further, among the apparatuses for transporting an indwelling device such as the stent graft (indwelling medical device transporting apparatus), there is also an apparatus that bends a tip portion of the sheath by using an operating line (not shown) in which an end portion on the distal side is fixed to the tip portion of the sheath and towing the operating line. Then, the stent graft inside the sheath is placed by pulling the sheath to the proximal side with the tip portion of the sheath being bent.

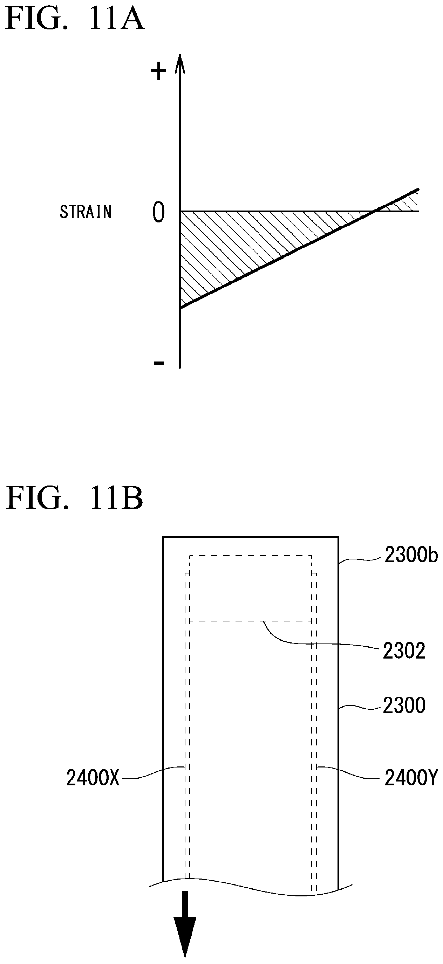

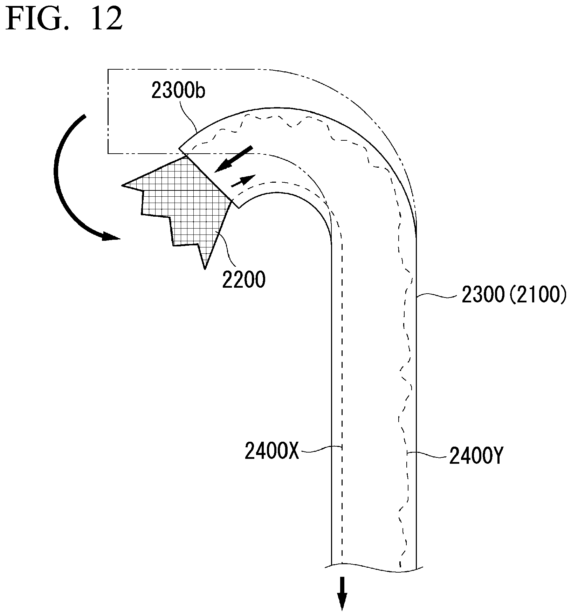

For example, as shown in FIGS. 11A and 11B, and FIG. 12, there is also a transporting apparatus 2100 that bends a tip portion 2300b of a sheath 2300 by towing one of a first operating line 2400X and a second operating line 2400Y.

FIG. 11A is a diagram schematically showing the amount of strain of the sheath 2300 when only the first operating line 2400X is towed. FIG. 11B is a side view showing a configuration of the tip portion 2300b of the sheath 2300. FIG. 12 is an explanatory diagram illustrating a load applied to the sheath 2300 when the sheath 2300 is retracted after the tip portion 2300b of the sheath 2300 is bent in the conventional transporting apparatus 2100.

More specifically, the first operating line 2400X and the second operating line 2400Y have their end portions on the distal sides fixed to the tip portion 2300b of the sheath 2300 (more specifically, a ring 2302 embedded in the sheath 2300). Further, the first operating line 2400X is connected to one side in the radial direction of the tip portion 2300b of the sheath 2300, and the second operating line 2400Y is connected to the other side on the opposite side thereof.

By selectively towing one of the first operating line 2400X and the second operating line 2400Y, an eccentric compressive load is applied to the tip portion 2300b to result in the amount of strain shown in FIG. 11A, so that the tip portion 2300b is selectively bent.

By pulling the sheath 2300 to the proximal side with the tip portion 2300b of the sheath 2300 being bent, a stent graft 2200 (see FIG. 12) inside the sheath 2300 is exposed from the sheath 2300 and placed.

### [Citation List]

### [Patent Document]

[[Patent Document 1] Japanese Unexamined Patent Application, First Publication No. 2013-52282

### [Summary of Invention]

### [Technical Problem]

However, since the sheath of Patent Document 1 is pre-shaped into a curved shape, when it is inserted directly into a lumen (aorta) of a living body without using a guide wire, the tip of the sheath always scratches a lumen wall (blood vessel wall) of the living body. For this reason, in the technique disclosed in Patent Document 1, an operation for passing the guide wire is required, and the operation becomes complicated.

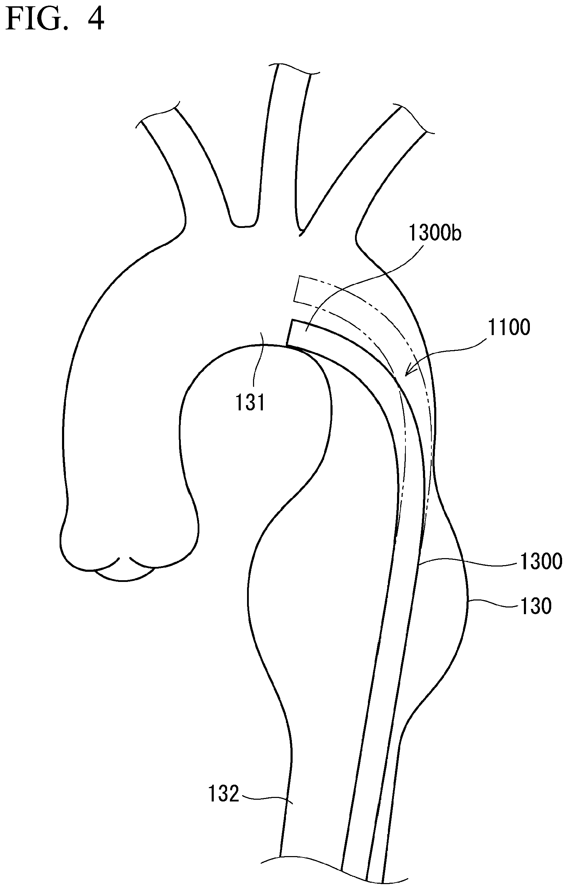

Further, an operator using a transporting apparatus 1100 shown in FIG. 4 having an operating line (not shown) bends and deforms a tip portion 1300b of a sheath 1300, so as to be aligned with the inner wall of, for example, a heavily curved aortic arch 131 or the like, by towing the operating line. Then the operator pulls the sheath 1300 to the proximal side in order to place an indwelling device such as a stent graft. At this time, as shown in FIG. 4, the tip portion 1300b of the sheath 1300 may move to the proximal side while being bent, and in this case, the stent graft expands to be placed at a position shifted from the center of the path of the aortic arch 131.

As described above, when the stent graft is expanded and placed at a position deviated from a desired position (for example, the center of the path of the aortic arch 131), it is assumed that a slight gap is generated between the stent graft and the lumen wall of the living body. In this case, blood may flow beyond the stent graft into a thoracic aortic aneurysm 130 formed in a descending aorta 132.

In order to deploy along the aortic arch 131, it is necessary to pull the sheath 1300 downward and gradually release the bending of the tip portion 1300b. However, skill is required for the operator to perform this operation, and it has also been difficult to perform this operation according to the manual.

Further, in the above-mentioned transporting apparatus 2100, even if the tip portion 2300b is set in the desired direction along the direction of the lumen of a living body, as shown in FIG. 12, when the sheath 2300 is retracted, the frictional force from the stent graft 2200 is applied to the tip portion 2300b of the sheath 2300, so that the tip portion 2300b may be further bent.

More specifically, when the tip portion 2300b of the sheath 2300 is bent, the first operating line 2400X on the bent side (inner circumferential side) becomes tense, but the first operating line 2400X on the opposite side thereof (outer circumferential side) remains relaxed. When the sheath 2300 is retracted in this state, the stent graft 2200 applies a biasing force toward the distal direction with respect to the tip portion 2300b of the sheath 2300 by the frictional force with the inner wall of the sheath 2300. This biasing force becomes a tensile load that extends the tip portion 2300b of the sheath 2300 on the outer circumferential side, and acts as a moment to bend the tip portion 2300b.

In this case, since the direction of the sheath 2300 at the time of setting is different, it has been difficult to place the stent graft 2200 at a position along the direction of the lumen of a living body.

Therefore, there has been a demand for a transporting apparatus capable of transporting an indwelling device (stent graft 2200) more accurately to an intended position in the lumen of a living body.

The present invention has been made in view of the above problems and provides: an indwelling medical device transporting apparatus capable of accurately placing an indwelling device at a suitable and intended position without performing complicated operations with respect to the apparatus; and an indwelling medical device transporting apparatus with an indwelling device.

### [Solution to Problem]

The indwelling medical device transporting apparatus of the present invention includes: a long and flexible tubular main body that holds an indwelling medical device to be placed in a body; an operating line slidably inserted into the aforementioned tubular main body and whose distal side is connected to a distal portion of the aforementioned tubular main body; an operating portion that is connected to a proximal side of the aforementioned operating line and bends the aforementioned distal portion of the aforementioned tubular main body by towing the aforementioned operating line; a restricting member capable of restricting movement of the aforementioned indwelling device to the proximal side; and a traction force changing portion that changes a traction force of the aforementioned tubular main body when relative positions of the aforementioned restricting member and the aforementioned tubular main body are changed so that the aforementioned tubular main body is relatively located on the proximal side, wherein the aforementioned traction force changing portion changes a traction force of the aforementioned operating line so as to suppress deflection of the aforementioned distal portion of the aforementioned tubular main body caused by changing the relative positions of the aforementioned restricting member and the aforementioned tubular main body.

Further, the indwelling medical device transporting apparatus with an indwelling device of the present invention includes the above-mentioned indwelling medical device transporting apparatus and an indwelling device held in a tubular main body.

Furthermore, the indwelling medical device transporting apparatus of the present invention includes: a long and flexible tubular main body that holds an indwelling medical device to be placed in a body; an operating line slidably inserted into the aforementioned tubular main body and whose distal side is connected to a distal portion of the aforementioned tubular main body; an operating portion that is connected to a proximal side of the aforementioned operating line and bends the aforementioned distal portion of the aforementioned tubular main body by towing the aforementioned operating line; a restricting member capable of restricting movement of the aforementioned indwelling device on the proximal side; and a rigidity imparting portion that imparts rigidity to the aforementioned tubular main body, wherein the aforementioned operating line is connected to at least one side in the radial direction in the aforementioned distal portion of the aforementioned tubular main body, and the aforementioned rigidity imparting portion increases rigidity of the other side on the opposite side of the aforementioned one side in the radial direction in the aforementioned distal portion of the aforementioned tubular main body.

Further, the indwelling medical device transporting apparatus with an indwelling device of the present invention includes the aforementioned indwelling device held in the aforementioned tubular main body.

### [Advantageous Effects of Invention]

According to the indwelling medical device transporting apparatus of the present invention, an indwelling device can be accurately placed at a suitable and intended position by changing the bent state of the distal portion of the tubular main body when relative positions of the restricting member and the tubular main body are changed.

Further, according to the indwelling medical device transporting apparatus with an indwelling device of the present invention, since the indwelling device is held in advance, the operation is facilitated.

### [Brief Description of Drawings]

FIG. 1 is a schematic view showing a state in which a stent graft is deployed by a transporting apparatus according to a first embodiment.
FIG. 2A is a diagram for explaining a mechanism inside a housing with an upper lid of the housing being removed, and shows a state before operation.
FIG. 2B is a diagram for explaining the mechanism inside the housing with the upper lid of the housing being removed, and shows a state in which an operating lever is operated and the operating lever is locked by a lock portion.
FIG. 2C is a diagram for explaining the mechanism inside the housing with the upper lid of the housing being removed, and shows a state in which a movement mechanism is operated.
FIG. 3 is a schematic view showing a state in which both end portions of a stent graft are arranged on an aortic arch and a descending aorta, and the stent graft is placed so as to straddle a thoracic aortic aneurysm.
FIG. 4 is a schematic view showing an action of a tip portion of a sheath in a conventional indwelling device transporting apparatus.
FIG. 5 is a schematic view showing a state in which a stent graft is deployed by a transporting apparatus according to a second embodiment.
FIG. 6A is a diagram for explaining a mechanism inside a housing with an upper lid of the housing being removed, and shows a state before operation.
FIG. 6B is a diagram for explaining the mechanism inside the housing with the upper lid of the housing being removed, and shows a state in which an operating slider is locked by a stopper with the operating slider being pulled to a proximal side.
FIG. 6C is a diagram for explaining the mechanism inside the housing with the upper lid of the housing being removed, and shows a state in which a movement mechanism is operated.
FIG. 7 is a schematic view showing a state in which both end portions of a stent graft are arranged on an aortic arch and a descending aorta, and the stent graft is placed so as to straddle a thoracic aortic aneurysm.
FIG. 8 is an explanatory diagram illustrating a load applied to a sheath when the sheath is retracted while maintaining a bent state of a tip portion of the sheath.
FIG. 9 is a cross sectional view of a tip portion of a sheath in which a coil member according to a third embodiment is embedded.
FIG. 10 is a perspective view showing a coil member.
FIG. 11A is a diagram schematically showing the amount of strain of the sheath when only a first operating line is towed.
FIG. 11B is a side view showing a configuration of the tip portion of the sheath.
FIG. 12 is an explanatory diagram illustrating a load applied to the sheath when the sheath is retracted after the tip portion of the sheath is bent in a conventional transporting apparatus.

### [Description of Embodiments]

Hereinafter, embodiments of the present invention will be described with reference to the drawings. The embodiments described below are merely examples for a better understanding of the present invention, and do not limit the present invention. In other words, the shapes, sizes, arrangements, and the like of the members described below can be changed or improved without departing from the gist of the present invention, and it goes without saying that the present invention includes equivalents thereof.

Further, in all the drawings, the same reference numerals are given to the same constituent elements, and duplicated descriptions thereof will be appropriately omitted.

### << First embodiment>>

### <Overview>

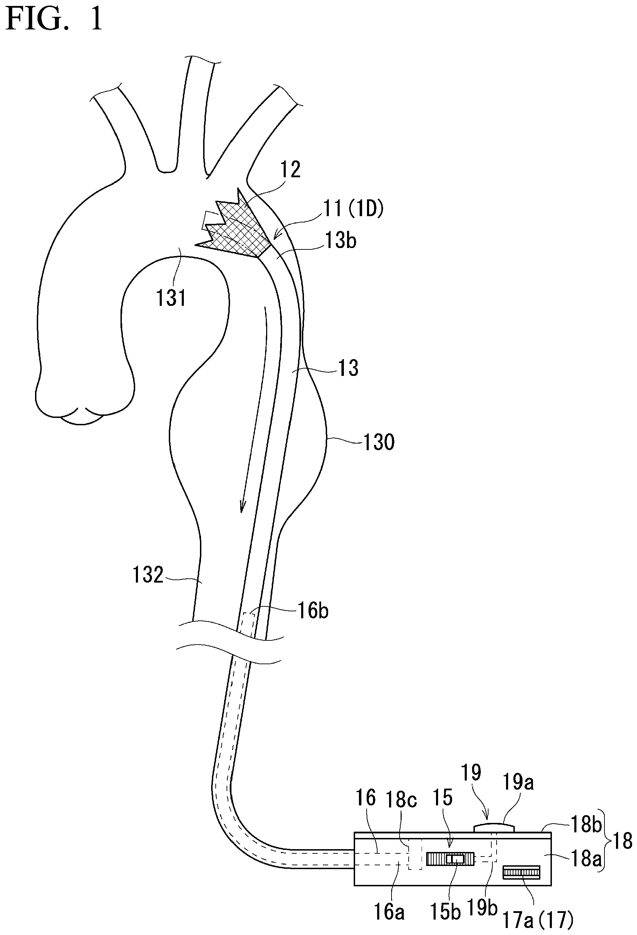

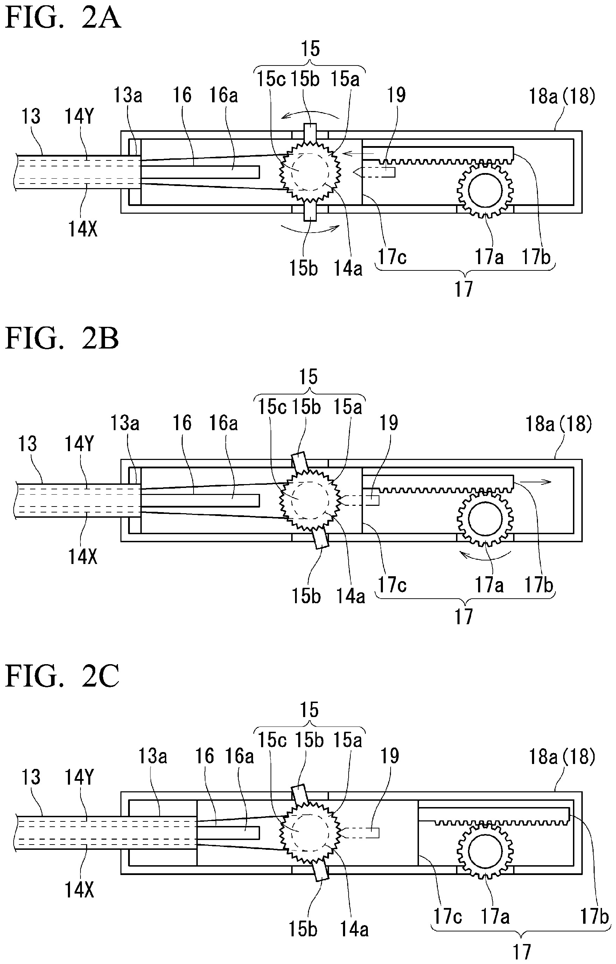

First, an outline of an indwelling medical device transporting apparatus (transporting apparatus 11) according to a first embodiment will be described with reference to FIGS. 1 and 2A to 2C. FIG. 1 is a schematic view showing a state in which a stent graft 12 is deployed by the transporting apparatus 11 according to the first embodiment. FIG. 2A is a diagram for explaining a mechanism inside a housing 18 with an upper lid 18b of the housing 18 being removed, and shows a state before operation. FIG. 2B is a diagram for explaining the mechanism inside the housing 18 with the upper lid 18b of the housing 18 being removed, and shows a state in which an operating lever 15 is operated and the operating lever 15 is locked by a lock portion 19. FIG. 2C is a diagram for explaining the mechanism inside the housing 18 with the upper lid 18b of the housing 18 being removed, and shows a state in which a movement mechanism 17 is operated.

The operating lever 15 and the movement mechanism 17 and the housing 18 including the operating lever 15 and the movement mechanism 17 in FIG. 1 are shown smaller than the actual ones for convenience of illustration and explanation.

The transporting apparatus 11 according to the present embodiment includes: a long and flexible tubular main body (sheath 13) that holds an indwelling medical device (stent graft 12) to be placed in a body; operating lines 14X and 14Y that are slidably inserted into the tubular main body (sheath 13) and whose distal sides are connected to a distal portion (tip portion 13b) of the tubular main body (sheath 13); an operating portion (operating lever 15) that is connected to a proximal side 14a of the operating lines 14X and 14Y and bends the distal portion (tip portion 13b) of the tubular main body (sheath 13) by towing the operating lines 14X and 14Y; a restricting member (holding shaft 16) capable of restricting movement of the indwelling device (stent graft 12) to the proximal side; and a traction force changing portion (movement mechanism 17) that changes a traction force of the tubular main body (sheath 13) when relative positions of the restricting member (holding shaft 16) and the tubular main body (sheath 13) are changed so that the tubular main body (sheath 13) is relatively located on the proximal side.

The traction force changing portion (movement mechanism 17) changes a traction force of the operating lines 14X and 14Y so as to suppress deflection of the distal portion (tip portion 13b) of the tubular main body (sheath 13) caused by changing the relative positions of the restricting member (holding shaft 16) and the tubular main body (sheath 13).

According to the above configuration, when relative positions of the restricting member (holding shaft 16) and the tubular main body (sheath 13) are changed due to change of the traction force of the operating lines 14X and 14Y by the traction force changing portion (movement mechanism 17), the deflection of the distal portion (tip portion 13b) of the tubular main body (sheath 13) can be automatically suppressed. For this reason, an indwelling device (stent graft 12) can be placed at a suitable position by changing a bent state of the distal portion (tip portion 13b) of the tubular main body (sheath 13) without performing complicated operations with respect to the apparatus.

The above-mentioned "indwelling device" includes, in addition to the stent graft 12, a stent, a prosthetic valve or another indwelling device to be placed in the lumen of a living body.

The above-mentioned "bending" includes, in addition to a sharply bent state, a curvature as a gently bent state.

The above expression "the proximal side 14a of the operating lines 14X and 14Y is connected" is not limited to a state in which "the proximal side 14a of the operating lines 14X and 14Y is fixed". For example, also included are those in which the movement of the operating lines 14X and 14Y in the traction direction is restricted by being wound around the operating portion (winding shaft 15c of the operating lever 15) in addition to the rotational action of the operating lever 15.

The above-mentioned "deflection" refers to a deviation of occupied areas in the lumen of a living body with respect to the accommodating portion of the stent graft 12 of the sheath 13 before and after the change in the relative positions of the holding shaft 16 and the sheath 13. Therefore, when the sheath 13 is ideally retracted by completely tracing inside the occupied area before the relative position change, the accommodating portions overlap before and after the relative position change, and there will be substantially no "deflection".

On the other hand, as described with reference to FIG. 4, when the sheath 13 is simply moved to the proximal side, there is a large deviation in the occupied area with respect to the accommodating portion, and the "deflection" will be large.

That is, by reducing the amount of bending of the tip portion 13b of the sheath 13 so that during the relative position change the distal end of the sheath 13 passes through inside the occupied area of the sheath 13 before the relative position change described above, the position overlapping or in the vicinity of the above area, the deflection of the tip portion 13b of the sheath 13 is suppressed. As a result, for example, it becomes easy to arrange the stent graft 12 in the vicinity of an intended position to be placed.

The change of the traction force by the traction force changing portion described above refers more specifically to weakening of the traction in conjunction with the relative retraction of the sheath 13 with respect to the holding shaft 16 from a state in which one of the operating lines 14X and 14Y is towed.

Further, as described above, in addition to a configuration in which the sheath 13 is retracted to retract relative to the holding shaft 16, a configuration in which the holding shaft 16 is moved forward to retract the sheath 13 relative to the holding shaft 16 is regressed is also conceivable.

Also in this case, since the relative positions of the sheath 13 and the holding shaft 16 are changed in the same manner, the traction force of the operating lines 14X and 14Y may be weakened so that the sheath 13 is retracted by tracing inside the occupied area of the sheath 13 before the relative movement, while the holding shaft 16 is moved forward relative to the sheath 13.

Further, the traction force of the operating lines 14X and 14Y may be adjusted by an electrical means.

Details of this means will be described later.

According to the transporting apparatus 11 according to the present embodiment, for example, when the sheath 13 or the holding shaft 16 is moved in a direction in which the sheath 13 is retracted relative to the holding shaft 16, it is possible to suppress the stent graft 12 from deploying at a position shifted from the center of the path of the aortic arch 131.

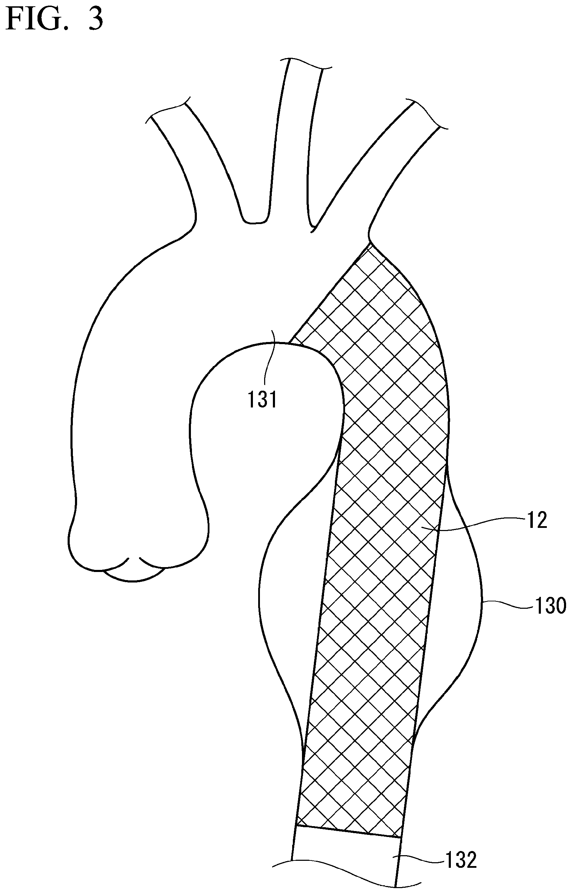

That is, as shown in FIG. 3, since the stent graft 12 can be arranged over the aortic arch 131 and the descending aorta 132 so as to straddle the thoracic aortic aneurysm 130, it is possible to prevent the blood from flowing into the thoracic aortic aneurysm 130.

FIG. 3 is a schematic view showing a state in which both end portions of the stent graft 12 are arranged on the aortic arch 131 and the descending aorta 132, and the stent graft 12 is placed so as to straddle the thoracic aortic aneurysm 130.

### <Details of each part of transporting apparatus>

Details of each part of the transporting apparatus 11 according to the present embodiment will be described with reference to FIGS. 1 and 2A to 2C.

As described above, the transporting apparatus 1 includes the sheath 13, the operating lines 14X and 14Y, the operating lever 15 that tows the operating lines 14X and 14Y and bends the tip portion 13b of the sheath 13, the holding shaft 16 capable of restricting movement of the stent graft 12 to the proximal side, and the movement mechanism 17 that changes a traction force of the sheath 13 when relative positions of the holding shaft 16 and the sheath 13 are changed so that the sheath 13 is relatively located on the proximal side.

In the sheath 13, a main lumen (not shown) accommodating the stent graft 12 and two sub-lumens (not shown) having a diameter smaller than that of the main lumen and having operating lines 14X and 14Y inserted therein are formed.

A thermoplastic polymer material can be used as the material of the sheath 13. Examples of the thermoplastic polymer material include polyimide (PI), polyamideimide (PAI), polyethylene terephthalate (PET), polyethylene (PE), polyamide (PA), polyamide elastomers (PAE), nylon elastomers such as polyether block amide (PEBA), polyurethane (PU), ethylene-vinyl acetate resins (EVA), polyvinyl chloride (PVC) or polypropylene (PP).

A metal mesh or the like as a reinforcing layer may be contained inside the sheath 13, and a tubular member may be embedded as a portion defining a main lumen or a sub-lumen.

Each of the distal end portions of the operating lines 14X and 14Y is fixed, for example, on the opposite sides to each other by sandwiching the shaft center of the sheath 13 in a ring-shaped member embedded in the tip portion 13b of the sheath 13. With this configuration, since a tensile force and a moment due to the tensile force are applied to the sheath 13 at a position eccentric to the shaft center of the sheath 13 when one of the operating lines 14X and 14Y is towed to the proximal side, it is possible to bend the tip portion 13b.

In the present embodiment, the operating lines 14X and 14Y are composed of two lines, but may be composed of more lines than these, or may be composed of one line in which end portions are arranged on both sides of the tip portion 13b in the radial direction.

It may be configured to be arranged only on one side in the radial direction of the tip portion 13b of the sheath 13, that is, only the operating line 14X or the operating line 14Y is arranged.

The operating lever 15 changes the bent state of the sheath 13, and the proximal side 14a of the operating line 14X and the operating line 14Y is wound around.

More specifically, the operating lever 15 includes: a roller portion 15a; a lever portion 15b that can be operated by an operator and extends from the roller portion 15a on both sides in the width direction; and a winding shaft 15c provided inside the roller portion 15a and to which the proximal side 14a of the operating lines 14X and 14Y is fixed.

When the operating lever 15 is rotated clockwise in FIGS. 2A to 2C, the operating line 14X relaxes and the operating line 14Y becomes tense, and the tip portion 13b of the sheath 13 can be bent to one side.

Conversely, when the operating lever 15 is rotated counterclockwise in FIGS. 2A to 2C, the operating line 14X becomes tense and the operating line 14Y relaxes, and the tip portion 13b of the sheath 13 can be bent to a side opposite to the one side.

The holding shaft 16 has a function of restricting the retraction of the stent graft 12 by disposing a tip portion 16b of the holding shaft 16 so as to be able to come into contact with a base end of the stent graft 12 accommodated in the sheath 13. A large part of the holding shaft 16 is accommodated in the sheath 13. Abase end portion 16a of the holding shaft 16 is accommodated in the housing 18, and the movement in the retraction direction is restricted by a restricting piece 18c provided on the upper lid 18b.

From a state in which the operating portion (operating lever 15) tows the operating lines 14X and 14Y to bend the distal portion (tip portion 13b) of the tubular main body (sheath 13), the tubular main body (sheath 13) is moved in the retraction direction relative to the restricting member (holding shaft 16), and the traction force changing portion (movement mechanism 17) reduces the traction force of the operating lines 14X and 14Y during this relative movement.

When the tubular main body (sheath 13) moves in the retraction direction with respect to the restricting member (holding shaft 16), the distal portion (tip portion 13b) of the tubular main body (sheath 13) tends to deflect in the retraction direction.

According to the above configuration, the traction force changing portion (movement mechanism 17) can suppress the deflection by reducing the traction force of the operating lines 14X and 14Y and reducing the amount of bending of the distal portion (tip portion 13b).

For example, the "traction force changing portion" is a member connected to the proximal side of the operating lines 14X and 14Y, and is not limited to those via the operating lever 15, but may be those in which the traction force is changed by the hand of an operator with respect to this member.

In addition, it may be a control unit (not shown) that electrically controls a separation mechanism (not shown) so as to separate (or cut) the operating lines 14X and 14Y from the lever in response to a signal indicating by a timer (not shown) that the tubular main body (sheath 13) has started to move in the retraction direction, and a predetermined time has elapsed.

The transporting apparatus 11 according to the present embodiment includes the movement mechanism 17 that moves the tubular main body (sheath 13) relative to the restricting member (holding shaft 16) in the retraction direction. The movement mechanism 17 functions as a traction force changing portion by moving the tubular main body (sheath 13) in the retraction direction, bringing the distal side of the operating lines 14X and 14Y closer to the proximal side 14a of the operating lines 14X and 14Y, and reducing the traction force of the operating lines 14X and 14Y.

According to the above configuration, by retracting the tubular main body (sheath 13) relative to the restricting member (holding shaft 16) by the movement mechanism 17, it is possible to reduce the distance between the distal side and the proximal side 14a of the operating lines 14X and 14Y and to reduce the traction force by weakening the tension of the operating lines 14X and 14Y.

The movement mechanism 17 is configured to operate independently of the operating lever 15. The movement mechanism 17 is composed of a rotatable dial 17a pivotally supported by the housing 18, a rack portion 17b that engages with the peripheral surface of the dial 17a, and an interstitial material 17c interposed between the tip side of the rack portion 17b and the base end portion 13a of the sheath 13.

The dial 17a is pivotally supported by a housing main body 18a, has teeth on the peripheral surface, and is configured to be rotatable by the operator by being partially exposed from the housing 18.

The rack portion 17b engages with the teeth provided on the peripheral surface of the dial 17a so as to move in the perspective direction in response to the rotation of the dial 17a.

The interstitial material 17c is a member that connects the sheath 13 and the rack portion 17b in order to move the sheath 13 in response to the movement of the rack portion 17b.

The operator rotates the dial 17a of the movement mechanism 17 clockwise as shown in FIG. 2B and moves the rack portion 17b to the proximal side when the sheath 13 is retracted with respect to the holding shaft 16 in order to place the stent graft 12. As shown in FIG. 2C, when the rack portion 17b moves to the proximal side, the sheath 13 also moves to the proximal side via the interstitial material 17c.

The movement mechanism 17 shown in FIGS. 1 and 2A to 2C conceptually shows the structure thereof, and it is preferable that the length of the rack portion 17b that engages with the teeth of the dial 17a is longer than the length of the stent graft 12.

However, considering that the stent graft 12 can spontaneously pop out from the sheath 13 due to its own elastic restoring force, the length of the rack portion 17b is not necessarily limited to those longer than the length of the stent graft 12.

As an example of the movement mechanism, in the present embodiment, one in which the sheath 13 is moved in the retraction direction relative to the holding shaft 16 by the movement mechanism 17 composed of the dial 17a, the rack portion 17b and the interstitial material 17c is explained. However, the movement mechanism according to the present invention is not limited to such a configuration, and for example, the sheath 13 may be moved by a movement mechanism such as a linear actuator provided with a ball screw or the like and electrically driven.

The transporting apparatus 11 according to the present embodiment includes the housing 18 that supports a part of the movement mechanism 17, and the operating portion (operating lever 15) is supported by the housing 18.

More specifically, the housing 18 according to the present embodiment is composed of the housing main body 18a and an upper lid 18b that covers the housing main body 18a. The housing 18 accommodates the base end portion 13a of the sheath 13, the roller portion 15a and the winding shaft 15c of the operating lever 15, the base end portion 16a of the holding shaft 16, and the movement mechanism 17.

The upper lid 18b is provided with the restricting piece 18c having a function of supporting the base end portion 16a of the holding shaft 16 and restricting the movement of the holding shaft 16 on the proximal side. The restricting piece 18c according to the present embodiment restricts the movement of the holding shaft 16 by coming into contact on the surface on the proximal side of the base end portion 16a, but is not limited to such a configuration if the movement can be restricted, and it may be configured so that the base end portion 16a is elastically held.

Since the operating lever 15 is supported by the housing 18, the operator can easily operate the movement mechanism 17 and the operating lever 15 around the housing 18.

The housing 18 supports the sheath 13 so as to restrict its movement in the rotational direction. For this reason, the operator can rotate the housing 18 together with the sheath 13 to perform a torque rotation operation of the sheath 13.

The transporting apparatus 11 according to the present embodiment includes a lock portion 19 that locks or unlocks an operation of the operating portion (operating lever 15).

The lock portion 19 is configured to include a slide operation portion 19a that can be slid in the perspective direction and a locking claw 19b that is connected to the slide operation portion 19a and locks the rotation of the roller portion 15a when coming into contact with the peripheral surface of the roller portion 15a. The slide operation portion 19a is supported by the upper lid 18b so as to be slidable in the perspective direction.

The operator brings the locking claw 19b into contact with the peripheral surface of the roller portion 15a and locks the rotation of the roller portion 15a by sliding the slide operation portion 19a to the distal side (the side where the locking claw 19b comes close to the roller portion 15a).

By doing so, the positions of the proximal side 14a of the operating lines 14X and 14Y wound (connected) to the lever portion 15b of the operating lever 15 can be fixed, and the bent state of the tip portion 13b of the sheath 13 to which the distal end portions of the operating lines 14X and 14Y are connected can be maintained at a stage before the sheath 13 is retracted.

An indwelling medical device transporting apparatus 1D with an indwelling device includes the above-mentioned indwelling medical device transporting apparatus (transporting apparatus 11) and an indwelling device (stent graft 12) held by a tubular main body (sheath 13).

More specifically, the stent graft 12 is accommodated in the main lumen of the sheath 13 in a state in which the diameter is reduced, and is held by the sheath 13 by the drag force from the wall surface defining the main lumen against the elastic restoring force of the stent graft 12 itself.

According to the indwelling medical device transporting apparatus 1D with an indwelling device, since the indwelling device (stent graft 12) is held in advance, the operation is facilitated.

In the above embodiment, although a configuration has been described in which the traction force of the operating lines 14X and 14Y is adjusted by the movement mechanism 17, the present invention is not limited to such a configuration.

For example, it may be a configuration including a strain gauge (not shown) that detects the amount of strain at the tip portion of the sheath 13, a traction force changing portion (a motor or the like for driving a roller having a winding shaft of the operating lines 14X, 14Y) that changes the traction force of the operating lines 14X and 14Y based on the detected amount of strain, and a control unit (not shown) for controlling the traction force changing portion.

More specifically, it may be configured so that: a strain gauge (not shown) is attached to the peripheral surface of the tip portion of the sheath 13; the control unit calculates the amount of strain from the signal detected by the strain gauge; and in response to the calculated amount of strain, a signal indicating the position in the perspective direction of the operating lever 15 to which the proximal side 14a of the operating lines 14X and 14Y is connected is transmitted to a traction force changing portion (not shown) to change the traction force to the sheath 13.

### << Second embodiment >>

### <Overview>

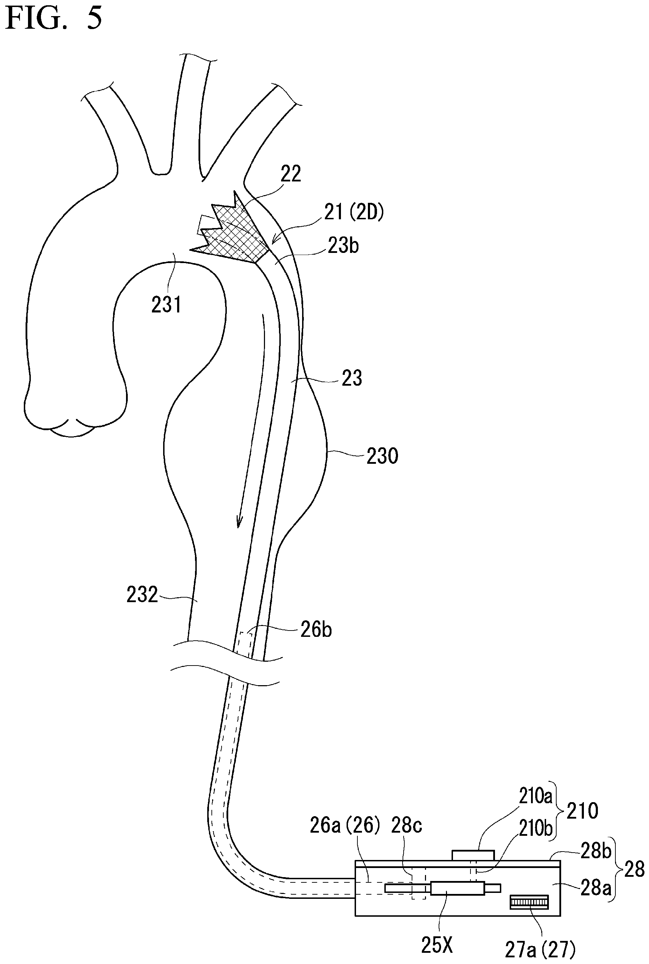

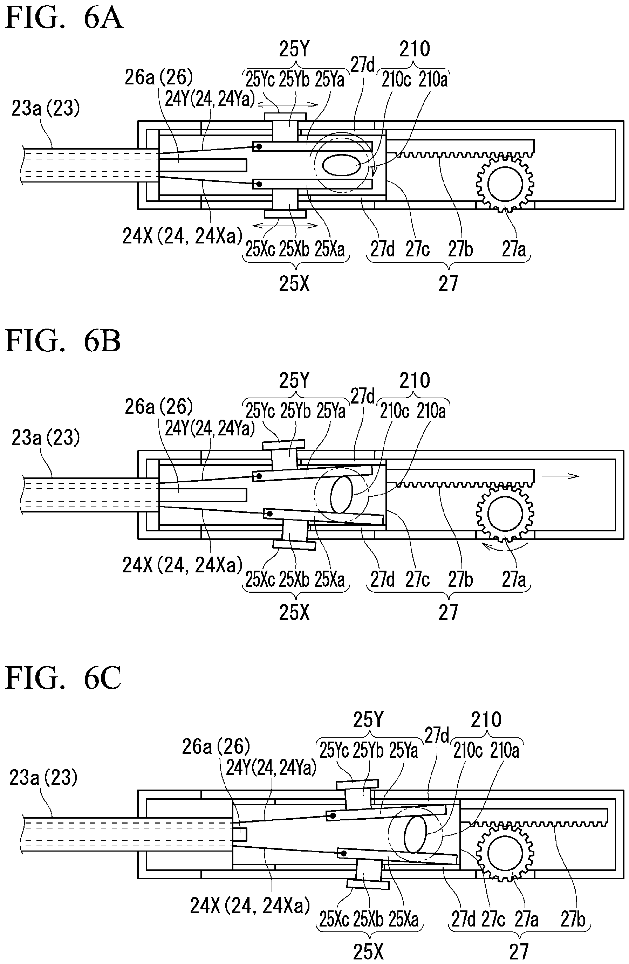

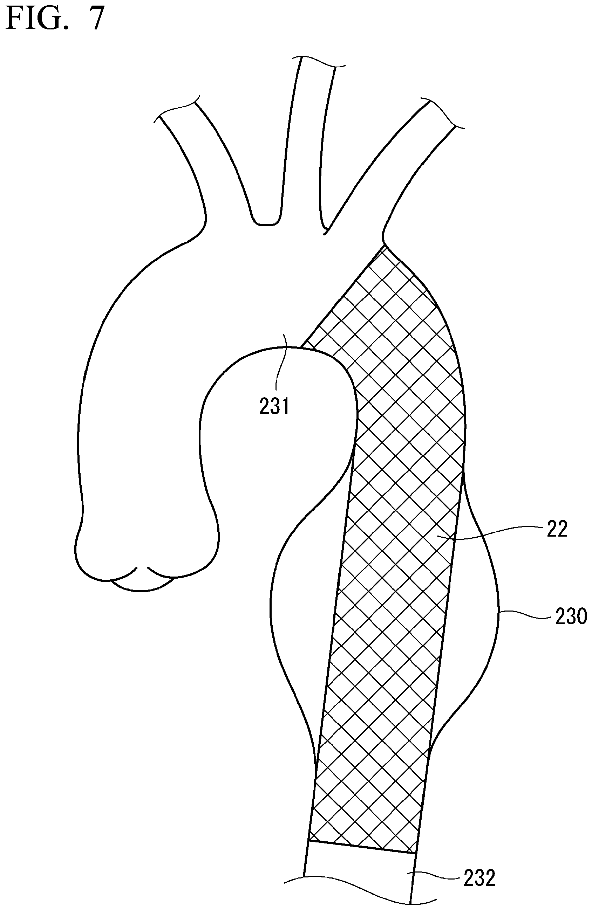

Next, an outline of an indwelling medical device transporting apparatus(transporting apparatus 21) according to a second embodiment will be described with reference to FIGS. 5 to 7. FIG. 5 is a schematic view showing a state in which a stent graft 22 is deployed by the transporting apparatus 21 according to the second embodiment. FIG. 6A is a diagram for explaining a mechanism inside a housing 28 with an upper lid 28b of the housing 28 being removed, and shows a state before operation. FIG. 6B is a diagram for explaining the mechanism inside the housing 28 with the upper lid 28b of the housing 28 being removed, and shows a state in which operating sliders 25X and 25Y are locked by a stopper 210 with the operating slider 25X being pulled to a proximal side. FIG. 6C is a diagram for explaining the mechanism inside the housing 28 with the upper lid 28b of the housing 28 being removed, and shows a state in which a movement mechanism 27 is operated. FIG. 7 is a schematic view showing a state in which both end portions of the stent graft 22 are arranged on an aortic arch 231 and a descending aorta 232, and the stent graft 22 is placed so as to straddle a thoracic aortic aneurysm 230.

The operating slider 25X and the movement mechanism 27 and the housing 28 including the operating slider 25X and the movement mechanism 27 in FIG. 5 are shown smaller than the actual ones for convenience of illustration and explanation.

The transporting apparatus 21 according to the present embodiment includes: a long and flexible tubular main body (sheath 23) that holds an indwelling medical device (stent graft 22) to be placed in a body; an operating line 24 slidably inserted into the tubular main body (sheath 23) and whose distal side is connected to a distal portion (tip portion 23b) of the tubular main body (sheath 23); an operating portion (operating slider 25X) that is connected to a proximal side 24Xa of the operating line 24 (first operating line 24X) and bends the distal portion (tip portion 23b) of the tubular main body (sheath 23) by towing the operating line 24 (first operating line 24X); a restricting member (holding shaft 26) capable of restricting movement of the indwelling device (stent graft 22) on the proximal side; and a rigidity imparting portion (second operating line 24Y) that imparts rigidity to the tubular main body (sheath 23).

The operating line 24 (first operating line 24X) is connected to at least one side in the radial direction in the distal portion (tip portion 23b) of the tubular main body (sheath 23). The rigidity imparting portion (second operating line 24Y) increases rigidity of the other side on the opposite side of the one side in the radial direction in the distal portion (tip portion 23b) of the tubular main body (sheath 23).

According to the above configuration, in a state in which the distal portion (tip portion 23b) of the tubular main body (sheath 23) is bent by the operating line 24 (first operating line 24X), the indwelling device (stent graft 22) can be suitably placed in the body by moving the tubular main body (sheath 23) relative to the restricting member (holding shaft 26).

More specifically, the rigidity of the tubular main body (sheath 23) is increased by the rigidity imparting portion (second operating line 24Y), and the frictional force is applied from the indwelling device (stent graft 22), as a result of which an increase in the amount of bending of the distal portion (tip portion 23b) of the tubular main body (sheath 23) can be suppressed (see FIG. 12).

For this reason, it becomes possible to place the indwelling device (stent graft 22) at a suitable position without performing complicated operations with respect to the transporting apparatus 21.

The above-mentioned "indwelling device" includes, in addition to the stent graft 22, a stent, a prosthetic valve or another indwelling device to be placed in the lumen of a living body.

The above-mentioned "bending" includes, in addition to a sharply bent state, a curvature as a gently bent state.

The above expression "the proximal side 24Xa of the operating line 24 is connected" is not limited to a state in which "the proximal side 24Xa of the operating line 24 is fixed". It may suffice as long as the movement of the operating line 24X in the traction direction can be restricted, and for example, the proximal side 24Xa of the operating line 24 may be wound and connected to a part of the operating portion.

The term "rigidity" according to the present embodiment refers to a property that is not distorted by a force in the bending direction.

The above-mentioned expression "imparting rigidity" includes, in addition to increasing the rigidity by providing a drag force (compressive force) against a load (tensile force) applied to the tip portion 23b of the sheath 23 in the present embodiment, increasing the rigidity by providing a member having a Young's modulus higher than those of the surroundings, which will be described later in the third embodiment.

According to the transporting apparatus 21 according to the present embodiment, for example, when the sheath 23 or the holding shaft 26 is moved in a direction in which the sheath 23 is retracted relative to the holding shaft 26, it is possible to suppress the stent graft 22 from deploying at a position shifted from the center of the path of the aortic arch 231.

That is, as shown in FIG. 7, since the stent graft 22 can be arranged over the aortic arch 231 and the descending aorta 232 so as to straddle the thoracic aortic aneurysm 230, it is possible to prevent the blood from flowing into the thoracic aortic aneurysm 230.

### <Details of each part of transporting apparatus>

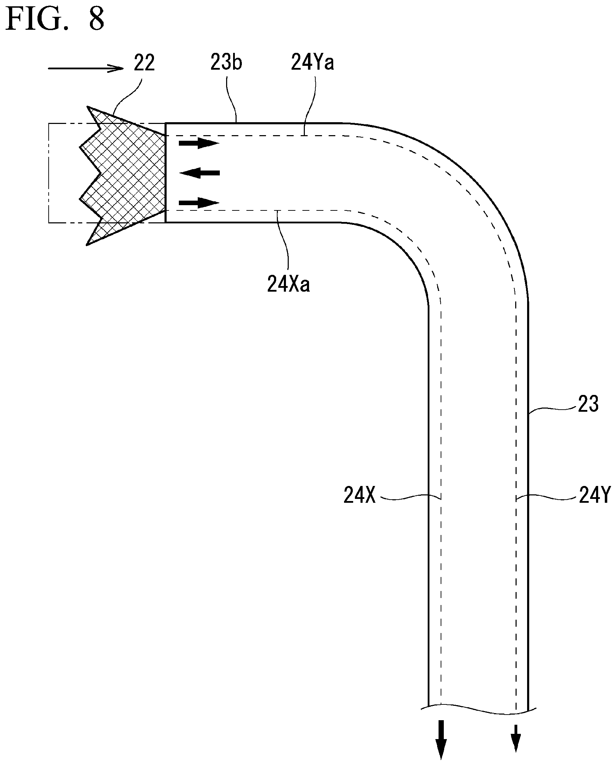

Details of each part of the transporting apparatus 21 according to the present embodiment will be described with reference mainly to FIG. 8 in addition to FIGS. 5 to 7. FIG. 8 is an explanatory diagram illustrating a load applied to the sheath 23 when the sheath 23 is retracted while maintaining a bent state of the tip portion 23b of the sheath 23.

As described above, the transporting apparatus 21 according to the present embodiment includes: the sheath 23; the first operating line 24X and the second operating line 24Y; the operating sliders 25X and 25Y that tow the first operating line 24X and the second operating line 24Y and bend the tip portion 23b of the sheath 23, or maintain the bent state of the tip portion 23b; the holding shaft 26 capable of restricting movement of the stent graft 22 to the proximal side; and the movement mechanism 27 that moves the sheath 23 so as to change relative positions of the holding shaft 26 and the sheath 23.

In the sheath 23, a main lumen (not shown) accommodating the stent graft 22 and two sub-lumens (not shown) having a diameter smaller than that of the main lumen and having the first operating line 24X and the second operating line 24Y inserted therein are formed.

A thermoplastic polymer material can be used as the material of the sheath 23. Examples of the thermoplastic polymer material include polyimide (PI), polyamideimide (PAI), polyethylene terephthalate (PET), polyethylene (PE), polyamide (PA), polyamide elastomers (PAE), nylon elastomers such as polyether block amide (PEBA), polyurethane (PU), ethylene-vinyl acetate resins (EVA), polyvinyl chloride (PVC) or polypropylene (PP).

A metal mesh or the like as a reinforcing layer may be contained inside the sheath 23, and a tubular member may be embedded as a portion defining a main lumen or a sub-lumen.

In particular, as described above, the transporting apparatus 21 according to the present embodiment further includes a second operating line 24Y that is provided separately from an operating line as the first operating line 24X and slidably inserted into the tubular main body (sheath 23), and whose distal side is connected to the opposite side of the tubular main body (sheath 23). The first operating line 24X and the second operating line 24Y are, for example, steel wires having a diameter of about 0.2 mm.

The second operating line 24Y functions as a rigidity imparting portion that imparts rigidity to the opposite side of a base end portion 23a of the sheath 23 by being towed by a traction force smaller than the traction force of the first operating line.

More specifically, as shown in FIG. 8, the second operating line 24Y increases rigidity by applying a load (compressive force) that acts as a drag force against a load (frictional force as well as being a tensile force) applied from the stent graft 22 to the tip portion 23b of the sheath 23 for further bending the tip portion 23b.

According to the above configuration, by providing a traction force by the second operating line 24Y which is smaller than that by the first operating line 24X, it is possible to suppress changes in the bending state of the sheath 23 before and after the retraction of the sheath 23 in the bending state.

In the present embodiment, there is no structural difference between the first operating line 24X and the second operating line 24Y.

That is, when the tip portion 23b of the sheath 23 is bent toward the traction direction side of the first operating line 24X, the second operating line 24Y on the other side can function as a rigidity imparting portion. On the other hand, when the tip portion 23b of the sheath 23 is bent toward the traction direction side of the second operating line 24Y, the first operating line 24X on the other side can function as a rigidity imparting portion.

Each of the distal end portions of the first operating line 24X and the second operating line 24Y is fixed, for example, on the opposite sides to each other by sandwiching the shaft center of the sheath 23 in a ring-shaped member embedded in the tip portion 23b of the sheath 23. This configuration is the same as that of each of the distal end portions of the first operating line 2400X and the second operating line 2400Y fixed to a ring 2302 shown in FIGS. 11A and 11B.

With this configuration, since a tensile force and a moment due to the tensile force are applied to the sheath 23 at a position eccentric to the shaft center of the sheath 23 when one of the first operating line 24X and the second operating line 24Y is towed to the proximal side, it is possible to bend the tip portion 23b.

In the present embodiment, the first operating line 24X and the second operating line 24Y are composed of two lines, but may be composed of more lines than these. Furthermore, depending on the configuration for holding the first operating line 24X and the second operating line 24Y, they may be composed by one line in which end portions are arranged on both sides of the tip portion 23b in the radial direction.

Although the details will be described later, it may be configured so that the rigidity imparting portion is provided separately from the operating line 24, and arranged only on one side in the radial direction of the tip portion 23b of the sheath 23, that is, only the first operating line 24X or the second operating line 24Y is arranged.

The rigidity imparting portion is configured to include a traction portion (operating slider 25Y) capable of towing the second operating line 24Y to the proximal side and to which the proximal side 24Ya of the second operating line 24Y is connected. The traction portion (operating slider 25Y) is configured to be operable independently of the operating portion (operating slider 25X).

According to the above configuration, it is easy to set the traction force of the first operating line 24X by the operating slider 25X (the force to bend the tip portion 23b of the sheath 23) and the traction force of the second operating line 24Y by the operating slider 25Y (the force to suppress deformation of the tip portion 23b of the sheath 23).

In the present embodiment, the operating slider 25X and the operating slider 25Y change or maintain the bent state of the sheath 23. There is no structural difference between them, and each is supported by the housing 28 so as to be slidable in the perspective direction.

More specifically, the operating slider 25X includes: an inner piece 25Xa arranged on the inner side of the housing 28 and to which the proximal side 24Xa of the first operating line 24X is connected; a middle piece 25Xb extending from the inner piece 25Xa to the outside on the side surface side of the housing 28; and an outer piece 25Xc that is arranged outside on the side surface side of the housing 28 and can be operated by the operator.

Similarly, the operating slider 25Y includes: an inner piece 25Ya arranged on the inner side of the housing 28 and to which the proximal side 24Ya of the second operating line 24Y is connected; a middle piece 25Yb extending from the inner piece 25Ya to the outside on the side surface side of the housing 28; and an outer piece 25Yc that is arranged outside on the side surface side of the housing 28 and can be operated by the operator.

When the tip portion 23b of the sheath 23 is bent toward the traction direction side of the first operating line 24X, the operating slider 25X functions as an operating portion and the operating slider 25Y functions as a traction portion, and when the tip portion 23b of the sheath 23 is bent toward the traction direction side of the second operating line 24Y, the operating slider 25X functions as a traction portion and the operating slider 25Y functions as an operating portion.

Although it is preferable if an operating slider 25Y (or operating slider 25X) as a traction portion is present because it is easy for the operator to grasp and operate, the stopper 210 is not necessarily limited to those provided with the operating slider 25Y (or operating slider 25X). That is, the operator may directly tow the second operating line 24Y (or the first operating line 24X) to impart rigidity to the tip portion 23b of the sheath 23.

The rigidity imparting portion is configured to include the traction portion (operating slider 25Y) and a traction holding portion (stopper 210) in which the second operating line 24Y can be directly or indirectly held by the tubular main body (sheath 23) while being towed by the traction portion (operating slider 25Y) in a state in which the tubular main body (sheath 23) is bent by the first operating line 24X.

According to the above configuration, the second operating line 24Y can be held by the operating slider 25Y and the stopper 210 in a state in which the tip portion 23b of the sheath 23 is pulled. When the stent graft 22 is placed, by applying a load in the opposite direction to the frictional force to the sheath 23 from the stent graft 22, it is possible to suppress an increase in the amount of bending of the tip portion 23b of the sheath 23.

More specifically, the traction holding portion (stopper 210) holds the second operating line 24Y in a towed state by pressing and holding the traction portion (operating slider 25Y) onto the tubular main body (sheath 23) or a member (side wall 27d of the interstitial material 27c) connected to the tubular main body (sheath 23).

By pressing and holding the stopper 210 onto the sheath 23 or the side wall 27d of the interstitial material 27c connected to the sheath 23, the operating slider 25Y can be interlocked with the operation of the sheath 23. For this reason, since the sheath 23 can be retracted in a state in which the traction force of the second operating line 24Y is kept constant, it is possible to suppress an increase in the amount of bending of the tip portion 23b of the sheath 23 when the sheath 23 is retracted.

More specifically, the stopper 210 has a stopper main body 210c capable of pressing the operating slider 25Y, and is supported by the housing 28 so as to be rotatable and slidable in the axial direction of the rack portion 27b.

The stopper main body 210c is formed in an elliptical shape having a size different from that in the radial direction from the center of rotation.

According to the above configuration, by rotating the stopper 210, it is possible to adjust the pressing state and the pressing release state by the operating slider 25Y with respect to the operating slider 25Y.

As an operation of the operating sliders 25X and 25Y, for example, from the state shown in FIG. 6A, by sliding the operating slider 25X to the proximal side, the operator bends the tip portion 23b of the sheath 23 to a side on which the first operating line 24X tows, thereby bringing the tip portion 23b into a bent state that matches the curvature of the aortic arch 231.

The operating slider 25Y is slid to the proximal side by a distance shorter than the operating slider 25X, and a tension by the second operating line 24Y is applied to the tip portion 23b of the sheath 23 to increase the rigidity of the tip portion 23b.

Furthermore, the operator rotates a knob 210a and presses the inner piece 25Xa of the operating slider 25X with the side wall 27d by the stopper main body 210c connected to the knob 210a via a connecting rod 210b while pressing the inner piece 25Ya of the operating slider 25Y with the side wall 27d.

In this manner, as a state shown in FIG. 6B, the bent state of the tip portion 23b of the sheath 23 is held by the stopper 210.

Each of the operating sliders 25X and 25Y is sandwiched between the side wall 27d of the interstitial material 27c and the stopper main body 210c of the stopper 210. For this reason, when the sheath 23 is moved to the proximal side by functioning the movement mechanism 27, as the rack portion 27b, the interstitial material 27c and the sheath 23 move to the proximal side, the operating sliders 25X and 25Y will also move to the proximal side. Therefore, the distance between both ends of the operating line 24 and the traction force in accordance with the distance do not substantially change before and after the operation of the movement mechanism 27, and the bent state of the tip portion 23b of the sheath 23 does not change.

The holding shaft 26 has a function of restricting the retraction of the stent graft 22 by disposing a tip portion 26b of the holding shaft 26 so as to be able to come into contact with a base end of the stent graft 22 accommodated in the sheath 23. A large part of the holding shaft 26 is accommodated in the sheath 23. A base end portion 26a of the holding shaft 26 is accommodated in the housing 28, and the movement in the retraction direction is restricted by a restricting piece 28c provided on the upper lid 28b.

The movement mechanism 27 according to the present embodiment has a function of moving the sheath 23 relative to the holding shaft 26 in the retraction direction. The movement mechanism 27 is composed of a rotatable dial 27a pivotally supported by the housing 28, a rack portion 27b that engages with the peripheral surface of the dial 27a, and an interstitial material 27c interposed between the tip side of the rack portion 27b and the base end portion 23a of the sheath 23.

The dial 27a is pivotally supported by a housing main body 28a, has teeth on the peripheral surface, and is configured to be rotatable by the operator by being partially exposed from the housing 28.

The rack portion 27b engages with the teeth provided on the peripheral surface of the dial 27a so as to move in the perspective direction in response to the rotation of the dial 27a.

The interstitial material 27c is a member that connects the sheath 23 and the rack portion 27b in order to move the sheath 23 in response to the movement of the rack portion 27b. Side walls 27d are provided to stand on both sides of the interstitial material 27c in the width direction. By sandwiching each of the inner pieces 25Xa and 25Ya of the operating sliders 25X and 25Y between the side walls 27d on both sides and the stopper main body 210c, the operating sliders 25X and 25Y are interlocked with the interstitial material 27c.

When retracting the sheath 23 with respect to the holding shaft 26 in order to place the stent graft 22, the operator rotates the dial 7a of the movement mechanism 27 clockwise as shown in FIG. 6B, and moves the rack portion 27b to the proximal side. As shown in FIG. 6C, when the rack portion 27b moves to the proximal side, the sheath 23 also moves to the proximal side via the interstitial material 27c.

The movement mechanism 27 shown in FIGS. 5 and 6A to 6C conceptually shows the structure thereof, and it is preferable that the length of the rack portion 27b that engages with the teeth of the dial 27a is longer than the length of the stent graft 22.

However, considering that the stent graft 22 can spontaneously pop out from the sheath 23 due to its own elastic restoring force, the length of the rack portion 27b is not necessarily limited to those longer than the length of the stent graft 22.

As an example of the movement mechanism, in the present embodiment, one in which the sheath 23 is moved in the retraction direction relative to the holding shaft 26 by the movement mechanism 27 composed of the dial 27a, the rack portion 27b and the interstitial material 27c is explained. However, the movement mechanism according to the present invention is not limited to such a configuration, and for example, the sheath 23 may be moved by a movement mechanism such as a linear actuator provided with a ball screw or the like and electrically driven.

The housing 28 supports a part of the operating sliders 25X and 25Y and the movement mechanism 27.

More specifically, the housing 28 according to the present embodiment is composed of the housing main body 28a and the upper lid 28b that covers the housing main body 28a. The housing 28 accommodates the base end portion 23a of the sheath 23, the inner pieces 25Xa and 25Ya of the operating sliders 25X and 25Y, a part of the middle pieces 25Xb and 25Yb, the base end portion 26a of the holding shaft 26, and the movement mechanism 27.

The upper lid 28b is provided with the restricting piece 28c having a function of supporting the base end portion 26a of the holding shaft 26 and restricting the movement of the holding shaft 26 on the proximal side. The restricting piece 28c according to the present embodiment restricts the movement of the holding shaft 26 by coming into contact on the surface on the proximal side of the base end portion 26a, but is not limited to such a configuration if the movement can be restricted, and it may be configured so that the base end portion 26a is elastically held.

Since a part of the operating sliders 25X and 25Y and the movement mechanism 27 are supported by the housing 28, the operator can easily operate the operating sliders 25X and 25Y and the movement mechanism 27 around the housing 28.

The housing 28 supports the sheath 23 so as to restrict its movement in the rotational direction. For this reason, the operator can rotate the housing 28 together with the sheath 23 to perform a torque rotation operation of the sheath 23.

According to the transporting apparatus 21 according to the present embodiment, it is possible to apply tension to the operating line (second operating line 24Y) on the outer circumferential side of the tip portion 23b of the sheath 23 to hold the operating line in a state where there is no slack. For this reason, when the indwelling device (stent graft 22) is extruded, changes in the bent state at the tip portion 23b of the sheath 23 can be suppressed, and the indwelling device (stent graft 22) can be placed in a desired bent shape.

An indwelling medical device transporting apparatus 2D with an indwelling device includes the above-mentioned indwelling medical device transporting apparatus (transporting apparatus 21) and an indwelling device (stent graft 22) held by a tubular main body (sheath 23).

More specifically, the stent graft 22 is accommodated in the main lumen of the sheath 23 in a state in which the diameter is reduced, and is held by the sheath 23 by the drag force from the wall surface defining the main lumen against the elastic restoring force of the stent graft 22 itself.

According to the indwelling medical device transporting apparatus 2D with an indwelling device, the operation is facilitated because the stent graft 22 is held in advance.

### << Third embodiment >>

### <Overview>

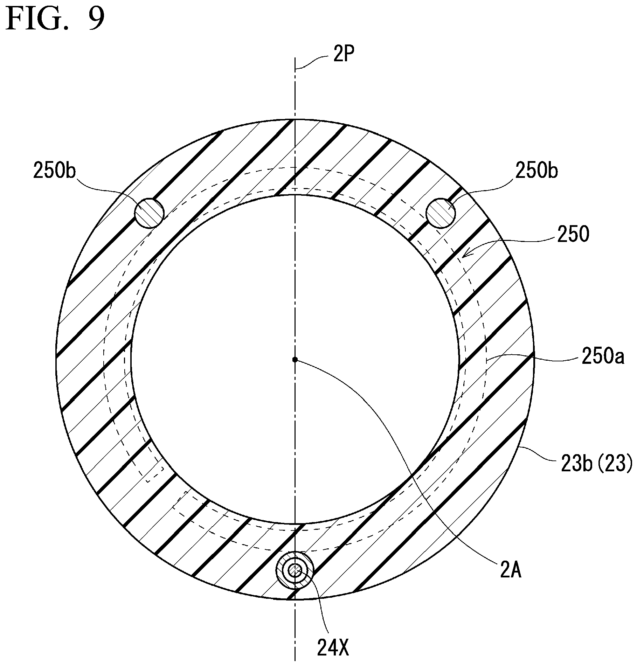

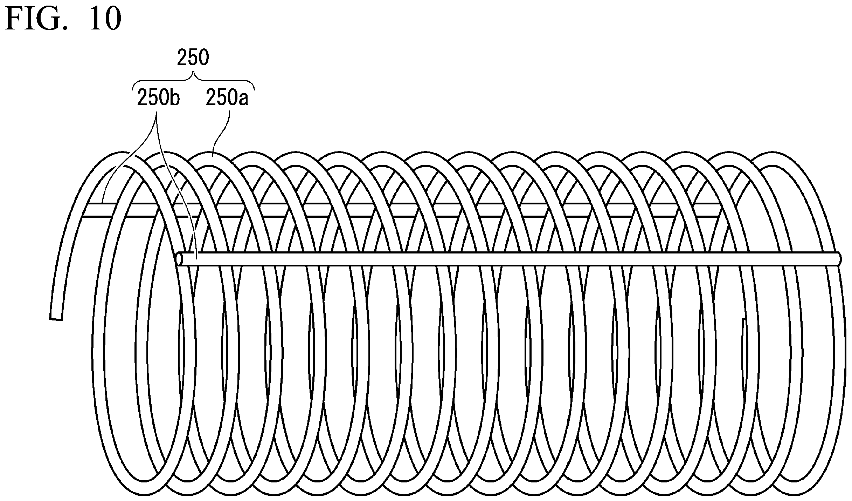

Next, an outline of an indwelling medical device transporting apparatus according to a third embodiment will be described with reference to FIGS. 9 and 10. FIG. 9 is a cross sectional view of the tip portion 23b of the sheath 23 in which a coil member 250 according to the third embodiment is embedded, and FIG. 10 is a perspective view showing the coil member 250.

FIG. 9 shows a cross section of a thick portion of the sheath 23, and the illustration of the stent graft 22 accommodated in an inner cavity of the sheath 23 is omitted.

The indwelling medical device transporting apparatus according to the present embodiment includes: an operating line (first operating line 24X) slidably inserted into a tubular main body (sheath 23) and whose distal side is connected to a distal portion (tip portion 23b) of the tubular main body (sheath 23); and a rigidity imparting portion (coil member 250) that imparts rigidity to the tubular main body (sheath 23).

The first operating line 24X is connected to one side in the radial direction in the distal portion (tip portion 23b) of the tubular main body (sheath 23).

The rigidity imparting portion (backbone portion 250b of the coil member 250) increases rigidity of the other side on the opposite side of the one side in the radial direction in the distal portion (tip portion 23b) of the tubular main body (sheath 23).

According to the above configuration, in a state in which the tip portion 23b of the sheath 23 is bent by the first operating line 24X, the stent graft 22 can be suitably placed in the body by retracting the sheath 23 relative to the holding shaft 26.

More specifically, the rigidity of the sheath 23 is increased by the backbone portion 250b of the coil member 250 having a higher Young's modulus than that of the surrounding resin material or the like, and the frictional force is applied from the stent graft 22, as a result of which an increase in the amount of bending of the tip portion 23b of the sheath 23 can be suppressed (see FIG. 12).

For this reason, it becomes possible to place the stent graft 22 at a suitable position without performing complicated operations with respect to the transporting apparatus.

The backbone portion 250b of the coil member 250 according to the present embodiment constantly increases the rigidity of the side opposite to the side to which the first operating line 24X is connected, particularly in the tip portion 23b of the sheath 23. For this reason, unlike the rigidity imparting portion according to the second embodiment by towing the second operating line 24Y, the rigidity imparting portion according to the present embodiment is preferable from the viewpoint that no special operation (traction) is required.

### <Details of each part of transporting apparatus>

The rigidity imparting portion (backbone portion 250b of the coil member 250) is at least a part (backbone portion 250b) of a reinforcing member (coil member 250) embedded in the tubular main body (sheath 23).

According to the above configuration, the coil member 250 can increase the rigidity of the opposite side than the one side and suppress the deflection (additional bending) of the sheath 23 when retracting the sheath 23.

The reinforcing member is a coil member 250 composed of a plurality of winding wires, and the coil member 250 includes a winding wire connecting portion (backbone portion 250b) that is fixed to the plurality of winding wires (coil main body 250a), arranged along the axial direction of the coil member 250, and connects at least a portion of the plurality of winding wires. The winding wire connecting portion (backbone portion 250b) is arranged on the opposite side of the tubular main body (sheath 23) and functions as a rigidity imparting portion.

Here, the phrase "plurality of winding wires" is arbitrary whether or not there are a plurality of winding wires, and the term "winding wire" means one loop of the spiral coil main body 250a.

According to the above configuration, the rigidity of the opposite side of the sheath 23 can be increased by the backbone portion 250b of the coil member 250, and the frictional force is applied from the stent graft 22, as a result of which an increase in the amount of bending of the distal portion of the sheath 23 can be suppressed.

In particular, since the coil member 250 is embedded in the tip portion 23b of the sheath 23, even if a part of the coil member 250 (curved winding wire portion) unexpectedly protrudes from the surface of the sheath 23, it is possible to suppress a local load from being applied to the living tissue in the body.

A plurality of winding wire connecting portions (backbone portions 250b) (two in the present embodiment) are provided. As shown in FIG. 9, the plurality of winding wire connecting portions (backbone portions 250b) are arranged symmetrically with respect to a virtual surface 2P including an operating line (first operating line 24X) inserted into the tubular main body (sheath 23) and an axis 2A of the tubular main body (sheath 23).

According to the above configuration, since the rigidity of the tip portion 23b of the sheath 23 can be increased in a wide range by the plurality of backbone portions 250b, it is also possible to suppress an uneven increase in the bending amount of the tip portion 23b of the sheath 23.

The reinforcing member according to the present invention is not limited to the coil member 250, and may be a so-called mesh-shaped metal member formed into a gridlike pattern in which a plurality of wires extending in two directions intersect in a circumferential direction, or it may be a resin material having a higher hardness (higher Young's modulus) than that of the surroundings.

Further, as a rigidity imparting portion according to the present invention, as a configuration for increasing the rigidity on the opposite side rather than the one side of the tip portion 23b of the sheath 23, in addition to a configuration in which a metal member (mesh or the like) is arranged only on the opposite side, a configuration in which there are more wires constituting a mesh on the opposite side than on the one side (a configuration having a high density) may be employed.

It is not necessary for the various components related to the transporting apparatus of the present invention to be individually and independently present. For example, it is possible for a plurality of components to be formed as a member, one component to be formed of a plurality of members, a certain component to be a part of another component, a part of a certain component to overlap a part of another component, or the like.

The above embodiments include the following technical ideas.
(1) An indwelling medical device transporting apparatus including: a long and flexible tubular main body that holds an indwelling medical device to be placed in a body;
   an operating line slidably inserted into the aforementioned tubular main body and whose distal side is connected to a distal portion of the aforementioned tubular main body;
   an operating portion that is connected to a proximal side of the aforementioned operating line and bends the aforementioned distal portion of the aforementioned tubular main body by towing the aforementioned operating line;
   a restricting member capable of restricting movement of the aforementioned indwelling device to the proximal side; and
   a traction force changing portion that changes a traction force of the aforementioned tubular main body when relative positions of the aforementioned restricting member and the aforementioned tubular main body are changed so that the aforementioned tubular main body is relatively located on the proximal side,
   wherein the aforementioned traction force changing portion changes a traction force of the aforementioned operating line so as to suppress deflection of the aforementioned distal portion of the aforementioned tubular main body caused by changing the relative positions of the aforementioned restricting member and the aforementioned tubular main body.
(2) The indwelling medical device transporting apparatus according to (1), wherein the aforementioned traction force changing portion reduces the traction force of the aforementioned operating line when the aforementioned tubular main body moves in a retraction direction relative to the aforementioned restricting member from a state in which the aforementioned operating portion tows the aforementioned operating line to bend the aforementioned distal portion of the aforementioned tubular main body.
(3) The indwelling medical device transporting apparatus according to (1) or (2), further including a movement mechanism that moves the aforementioned tubular main body relative to the aforementioned restricting member in a retraction direction,
   wherein the aforementioned movement mechanism functions as the aforementioned traction force changing portion by moving the aforementioned tubular main body in the retraction direction and brings the aforementioned distal side of the aforementioned operating line closer to the aforementioned proximal side of the aforementioned operating line to reduce the traction force of the aforementioned operating line.
(4) The indwelling medical device transporting apparatus according to (3), including a housing that supports a part of the aforementioned movement mechanism,
   wherein the aforementioned operating portion is supported by the aforementioned housing.
(5) The indwelling medical device transporting apparatus according to any one of (1) to (4), further including a lock portion that locks or unlocks an operation of the aforementioned operating portion.
(6) An indwelling medical device transporting apparatus with an indwelling device, including the indwelling medical device transporting apparatus according to any one of (1) to (5), and
   the aforementioned indwelling device held in the aforementioned tubular main body.
(7) An indwelling medical device transporting apparatus including: a long and flexible tubular main body that holds an indwelling medical device to be placed in a body;
   an operating line slidably inserted into the aforementioned tubular main body and whose distal side is connected to a distal portion of the aforementioned tubular main body;
   an operating portion that is connected to a proximal side of the aforementioned operating line and bends the aforementioned distal portion of the aforementioned tubular main body by towing the aforementioned operating line;
   a restricting member capable of restricting movement of the aforementioned indwelling device on the proximal side; and
   a rigidity imparting portion that imparts rigidity to the aforementioned tubular main body,
   wherein
   the aforementioned operating line is connected to at least one side in a radial direction in the aforementioned distal portion of the aforementioned tubular main body, and
   the aforementioned rigidity imparting portion increases rigidity of the other side on the opposite side of the one side in the radial direction in the aforementioned distal portion of the aforementioned tubular main body.
(8) The indwelling medical device transporting apparatus according to (7), further including a second operating line that is provided separately from the aforementioned operating line as a first operating line and slidably inserted into the aforementioned tubular main body, and whose distal side is connected to the aforementioned opposite side of the aforementioned tubular main body,
   wherein the aforementioned second operating line functions as the aforementioned rigidity imparting portion by being towed by a traction force smaller than the traction force of the aforementioned first operating line.
(9) The indwelling medical device transporting apparatus according to (8), wherein the aforementioned rigidity imparting portion is configured to include a traction portion capable of towing the aforementioned second operating line to the proximal side and to which the proximal side of the aforementioned second operating line is connected, and
   the aforementioned traction portion is configured to be operable independently of the aforementioned operating portion.
(10) The indwelling medical device transporting apparatus according to (9), wherein the aforementioned rigidity imparting portion is configured to include the aforementioned traction portion and a traction holding portion in which the aforementioned second operating line can be directly or indirectly held by the aforementioned tubular main body while being towed by the aforementioned traction portion in a state in which the aforementioned tubular main body is bent by the aforementioned first operating line.
(11) The indwelling medical device transporting apparatus according to (10), wherein the aforementioned traction holding portion holds the aforementioned second operating line in a towed state by pressing and holding the aforementioned traction portion onto the aforementioned tubular main body or a member connected to the aforementioned tubular main body.
(12) The indwelling medical device transporting apparatus according to (7), wherein the aforementioned rigidity imparting portion is at least a part of a reinforcing member embedded in the aforementioned tubular main body.
(13) The indwelling medical device transporting apparatus according to (12), wherein the aforementioned reinforcing member is a coil member composed of a plurality of winding wires,
   the aforementioned coil member includes a winding wire connecting portion that is fixed to the aforementioned plurality of winding wires, arranged along an axial direction of the aforementioned coil member, and connects at least a portion of the aforementioned plurality of winding wires, and
   the aforementioned winding wire connecting portion is arranged on the aforementioned opposite side of the aforementioned tubular main body and functions as the aforementioned rigidity imparting portion.
(14) The indwelling medical device transporting apparatus according to (13), wherein a plurality of the aforementioned winding wire connecting portions are provided, and
   a plurality of the aforementioned winding wire connecting portions are arranged symmetrically with respect to a virtual surface including the aforementioned operating line inserted into the aforementioned tubular main body and an axis of the aforementioned tubular main body.
(15) An indwelling medical device transporting apparatus with an indwelling device, including the indwelling medical device transporting apparatus according to any one of (7) to (14), and
   the aforementioned indwelling device held in the aforementioned tubular main body.

### [Industrial Applicability]

It is possible to provide: an indwelling medical device transporting apparatus capable of accurately placing an indwelling device at a suitable and intended position without performing complicated operations with respect to the apparatus; and an indwelling medical device transporting apparatus with an indwelling device.

### [Reference Signs List]

ID: Indwelling medical device transporting apparatus with indwelling device
11: Transporting apparatus (indwelling medical device transporting apparatus)
12: Stent graft (indwelling device)
13: Sheath (tubular main body)
13a: Base end portion
13b: Tip portion (distal portion)
14X, 14Y: Operating line
14a: Proximal side
15: Operating lever (operating portion)
15a: Roller portion
15b: Lever portion
15c: Winding shaft
16: Holding shaft (restricting member)
16a: Base end portion
16b: Tip portion
17: Movement mechanism (traction force changing portion)
17a: Dial
17b: Rack portion
17c: Interstitial material
18: Housing
18a: Housing main body
18b: Upper lid
18c: Restricting piece
19: Lock portion
19a: Slide operation portion
19b: Locking claw
130: Thoracic aortic aneurysm
131: Aortic arch
132: Descending aorta
1100: Transporting apparatus
1300: Sheath
1300b: Tip portion
2A: Axis
2D: Indwelling medical device transporting apparatus with indwelling device
2P: Virtual surface
21: Transporting apparatus (indwelling medical device transporting apparatus)
22: Stent graft (indwelling device)
23: Sheath (tubular main body)
23a: Base end portion
23b: Tip portion (distal portion)
24: Operating line
24X: First operating line
24Y: Second operating line (rigidity imparting portion)
24Xa, 24Ya: Proximal side
25X: Operating slider (operating portion)
25Y: Operating slider (traction portion)
25Xa, 25Ya: Inner piece
25Xb, 25Yb: Middle piece
25Xc, 25Yc: Outer piece
26: Holding shaft (restricting member)
26a: Base end portion
26b: Tip portion
27: Movement mechanism
27a: Dial
27b: Rack portion
27c: Interstitial material
27d: Side wall
28: Housing
28a: Housing main body
28b: Upper lid
28c: Restricting piece
210: Stopper (traction holding portion)
210a: Knob
210b: Connecting rod
210c: Stopper main body
230: Thoracic aortic aneurysm
231: Aortic arch
232: Descending aorta
250: Coil member (reinforcing member)
250a: Coil main body
250b: Backbone portion (winding wire connecting portion, rigidity imparting portion)
2100: Transporting apparatus
2200: Stent graft
2300: Sheath
2300b: Tip portion
2302: Ring
2400X: First operating line
2400Y: Second operating line

## Claims

1. An indwelling medical device transporting apparatus comprising:
a long and flexible tubular main body that holds an indwelling medical device to be placed in a body;
an operating line slidably inserted into said tubular main body and whose distal side is connected to a distal portion of said tubular main body;
an operating portion that is connected to a proximal side of said operating line and bends said distal portion of said tubular main body by towing said operating line;
a restricting member capable of restricting movement of said indwelling device to the proximal side; and
a traction force changing portion that changes a traction force of said tubular main body when relative positions of said restricting member and said tubular main body are changed so that said tubular main body is relatively located on the proximal side,
wherein said traction force changing portion changes a traction force of said operating line so as to suppress deflection of said distal portion of said tubular main body caused by changing the relative positions of said restricting member and said tubular main body.

2. The indwelling medical device transporting apparatus according to Claim 1, wherein said traction force changing portion reduces the traction force of said operating line when said tubular main body moves in a retraction direction relative to said restricting member from a state in which said operating portion tows said operating line to bend said distal portion of said tubular main body.

3. The indwelling medical device transporting apparatus according to Claim 1 or 2, further comprising a movement mechanism that moves said tubular main body relative to said restricting member in a retraction direction,
wherein said movement mechanism functions as said traction force changing portion by moving said tubular main body in the retraction direction and brings said distal side of said operating line closer to said proximal side of said operating line to reduce the traction force of said operating line.

4. The indwelling medical device transporting apparatus according to Claim 3, comprising a housing that supports a part of said movement mechanism,
wherein said operating portion is supported by said housing.

5. The indwelling medical device transporting apparatus according to any one of Claims 1 to 4, further comprising a lock portion that locks or unlocks an operation of said operating portion.

6. An indwelling medical device transporting apparatus with an indwelling device, comprising
the indwelling medical device transporting apparatus according to any one of Claims 1 to 5, and
said indwelling device held in said tubular main body.

7. An indwelling medical device transporting apparatus comprising:
a long and flexible tubular main body that holds an indwelling medical device to be placed in a body;
an operating line slidably inserted into said tubular main body and whose distal side is connected to a distal portion of said tubular main body;
an operating portion that is connected to a proximal side of said operating line and bends said distal portion of said tubular main body by towing said operating line;
a restricting member capable of restricting movement of said indwelling device on the proximal side; and
a rigidity imparting portion that imparts rigidity to said tubular main body,
wherein
said operating line is connected to at least one side in a radial direction in said distal portion of said tubular main body, and
said rigidity imparting portion increases rigidity of the other side on the opposite side of said one side in the radial direction in said distal portion of said tubular main body.

8. The indwelling medical device transporting apparatus according to Claim 7, further comprising a second operating line that is provided separately from said operating line as a first operating line and slidably inserted into said tubular main body, and whose distal side is connected to said opposite side of said tubular main body,
wherein said second operating line functions as said rigidity imparting portion by being towed by a traction force smaller than the traction force of said first operating line.

9. The indwelling medical device transporting apparatus according to Claim 8,
wherein said rigidity imparting portion is configured to include a traction portion capable of towing said second operating line to the proximal side and to which the proximal side of said second operating line is connected, and
said traction portion is configured to be operable independently of said operating portion.

10. The indwelling medical device transporting apparatus according to Claim 9, wherein said rigidity imparting portion is configured to include said traction portion and a traction holding portion in which said second operating line can be directly or indirectly held by said tubular main body while being towed by said traction portion in a state in which said tubular main body is bent by said first operating line.

11. The indwelling medical device transporting apparatus according to Claim 10, wherein said traction holding portion holds said second operating line in a towed state by pressing and holding said traction portion onto said tubular main body or a member connected to said tubular main body.

12. The indwelling medical device transporting apparatus according to Claim 7,
wherein said rigidity imparting portion is at least a part of a reinforcing member embedded in said tubular main body.

13. The indwelling medical device transporting apparatus according to Claim 12,
wherein said reinforcing member is a coil member composed of a plurality of winding wires,
said coil member includes a winding wire connecting portion that is fixed to said plurality of winding wires, arranged along an axial direction of said coil member, and connects at least a portion of said plurality of winding wires, and
said winding wire connecting portion is arranged on said opposite side of said tubular main body and functions as said rigidity imparting portion.

14. The indwelling medical device transporting apparatus according to Claim 13,
wherein a plurality of said winding wire connecting portions are provided, and
a plurality of said winding wire connecting portions are arranged symmetrically with respect to a virtual surface including said operating line inserted into said tubular main body and an axis of said tubular main body.

15. An indwelling medical device transporting apparatus with an indwelling device, comprising
the indwelling medical device transporting apparatus according to any one of Claims 1 to 14, and
said indwelling device held in said tubular main body.
